# EUROPEAN PATENT SPECIFICATION

(11) **EP 2 739 644 B1**
(45) Date of publication and mention of the grant of the patent: **30.03.2016**
(21) Application number: 12772437.5
(22) Date of filing: 06.08.2012
(51) Int. Cl.: C07K 14/46, C07K 14/47

(54) **PROTEINS WITH PRO-APOPTOTIC AND ANTITELOMERASE ACTIVITY ON CANCER CELLS, THEIR PREPARATION AND USE.**
PROTEINE MIT PRO-APOPTOTISCHER UND ANTITELOMERASE-WIRKUNG AUF KREBSZELLEN, IHRE HERSTELLUNG UND VERWENDUNG
PROTÉINES AYANT UNE ACTIVITÉ PRO-APOPTOTIQUE ET ANTI-TÉLOMÉRASE SUR DES CELLULES CANCÉREUSES, LEUR PRÉPARATION ET LEUR UTILISATION

(30) Priority: 05.08.2011 IT FI20110168
(43) Date of publication of application: 11.06.2014
(73) Proprietor: Ziche, Marina, 50144 Firenze (IT); Pazzagli, Mario, 50129 Firenze (IT); De Filippis, Vincenzo, 35126 Padova (IT); Garaci, Enrico, 00198 Roma (IT); Palmieri, Angelo Michele, 71015 San Nicandro Garganico (IT); Refosco, Fiorenzo, 36078 Valdagno (IT); Squeo, Maria, 71015 Sannicandro Garganico (IT); Montemurno, Concetta Antonia, 71015 Sannicandro Garganico (IT); Stanisci, Francesco, 71015 Sannicandro Garganico (IT); Granito, Mario Leonardo, 71015 Sannicandro Garganico (IT)
(72) Inventor: Ziche, Marina, 50144 Firenze (IT); Pazzagli, Mario, 50129 Firenze (IT); De Filippis, Vincenzo, 35126 Padova (IT); Garaci, Enrico, 00198 Roma (IT); Palmieri, Angelo Michele, 71015 San Nicandro Garganico (IT); Refosco, Fiorenzo, 36078 Valdagno (IT); Squeo, Maria, 71015 Sannicandro Garganico (IT); Montemurno, Concetta Antonia, 71015 Sannicandro Garganico (IT); Stanisci, Francesco, 71015 Sannicandro Garganico (IT); Granito, Mario Leonardo, 71015 Sannicandro Garganico (IT)
(74) Representative: Brighenti, Livio
(86) International application number: PCT/IB2012/054010
(87) International publication number: WO 2013/021339

(56) References cited:
- WO-A1-2007/085879
- WO-A1-2011/001403
- WO-A2-01/42462
- WO-A2-02/47697
- US-A1- 2005 069 530
- DONNINI SANDRA ET AL: "A novel protein from the serum of Python sebae, structurally homologous with type-[gamma] phospholipase A2 inhibitor, displays antitumour activity.", THE BIOCHEMICAL JOURNAL 1 DEC 2011 LNKD- PUBMED:21834793, vol. 440, no. 2, 1 December 2011 (2011-12-01), pages 251-262, XP008146482, ISSN: 1470-8728

### SEQUENCE LISTING

<110> ZICHE et al, Maria
<120> Proteine ad attività apoptotica e antitelomerasica su cellule cancerose, loro preparazione ed uso
<130> 11312PTWO
<150> FI2011A000168
   <151> 2011-08-05
<160> 9
<170> PatentIn version 3.5
<210> 1
   <211> 182
   <212> PRT
   <213> Python sebae
<220>
   <221> MISC_FEATURE
   <222> (38)..(38)
   <223> methylproline
<400> 1
   His Lys Cys Glu Ile Cys His Gly Phe Gly Asp Asp Cys Asp Gly Tyr
   1               5                   10                  15
   Glu Glu Glu Cys Pro Ser Pro Glu Asp Lys Cys Gly Lys Ile Leu Ile
               20                  25                  30
   Asp Ile Ala Leu Ala Xaa Val Ser Phe Arg Ala Thr His Lys Asn Cys
           35                  40                  45
   Phe Ser Ser Ser Ile Cys Lys Leu Gly Arg Val Asp Ile His Val Trp
       50                  55                  60
   Asp Gly Val Tyr Met Arg Gly Arg Thr Asn Cys Cys Asp Asn Asp Gln
   65                  70                  75                  80
   Cys Glu Asp Gln Pro Leu Pro Gly Leu Pro Leu Ser Leu Gln Asn Gly
                   85                  90                  95
   Leu Tyr Cys Pro Gly Ala Phe Gly Ile Phe Thr Glu Asp Ser Thr Glu
               100                 105                 110
   His Glu Val Lys Cys Arg Gly Thr Glu Thr Met Cys Leu Asp Leu Val
           115                 120                 125
   Gly Tyr Arg Gln Glu Ser Tyr Ala Gly Asn Ile Thr Tyr Asn Ile Lys
       130                 135                 140
   Gly Cys Val Ser Ser Cys Pro Leu Val Thr Leu Ser Glu Arg Gly His
   145                 150                 155                 160
   Glu Gly Arg Lys Asn Asp Leu Lys Lys Val Glu Cys Arg Glu Ala Leu
                   165                 170                 175
   Lys Pro Ala Ser Ser Asp
               180
<210> 2
   <211> 182
   <212> PRT
   <213> Python sebae
<400> 2
   His Lys Cys Glu Ile Cys His Gly Phe Gly Asp Asp Cys Asp Gly Tyr
   1               5                   10                  15
   Gln Glu Glu Cys Pro Ser Pro Glu Asp Arg Cys Gly Lys Ile Leu Ile
               20                  25                  30
   Asp Ile Ala Leu Ala Pro Val Ser Phe Arg Ala Thr His Lys Asn Cys
           35                  40                  45
   Phe Ser Ser Ser Ile Cys Lys Leu Gly Arg Val Asp Ile His Val Trp
       50                  55                  60
   Glu Gly Val Tyr Ile Arg Gly Arg Thr Asn Cys Cys Asp Asn Asp Gln
   65                  70                  75                  80
   Cys Glu Asp Gln Pro Leu Pro Gly Leu Pro Leu Ser Leu Gln Asn Gly
                   85                  90                  95
   Leu Tyr Cys Pro Gly Ala Phe Gly Ile Phe Thr Glu Asp Ser Thr Glu
               100                 105                 110
   His Glu Val Lys Cys Arg Gly Thr Glu Thr Met Cys Leu Asp Leu Val
           115                 120                 125
   Gly Tyr Arg Gln Glu Ser Tyr Ala Gly Asn Ile Thr Tyr Asn Ile Lys
       130                 135                 140
   Gly Cys Val Ser Ser Cys Pro Leu Val Thr Leu Ser Glu Arg Gly His
   145                 150                 155                 160
   Glu Gly Arg Lys Asn Asp Leu Lys Lys Val Glu Cys Arg Glu Ala Leu
                   165                 170                 175
   Lys Tyr Glu Ser Ser Asp
               180
<210> 3
   <211> 182
   <212> PRT
   <213> Python sebae
<400> 3
   His Lys Cys Glu Ile Cys His Gly Phe Gly Asp Asp Cys Asp Gly Tyr
   1               5                   10                  15
   Glu Glu Glu Cys Pro Ser Pro Glu Asp Lys Cys Gly Lys Ile Leu Ile
               20                  25                  30
   Asp Ile Ala Leu Ala Pro Val Ser Phe Arg Ala Thr His Lys Asn Cys
           35                  40                  45
   Phe Ser Ser Ser Ile Cys Lys Leu Gly Arg Val Asp Ile His Val Trp
       50                  55                  60
   Asp Gly Val Tyr Met Arg Gly Arg Thr Asn Cys Cys Asp Asn Asp Gln
   65                  70                  75                  80
   Cys Glu Asp Gln Pro Leu Pro Gly Leu Pro Leu Ser Leu Lys Asn Gly
                   85                  90                  95
   Leu Tyr Cys Pro Gly Ala Phe Gly Ile Phe Thr Glu Asp Ser Thr Glu
               100                 105                 110
   His Glu Val Lys Cys Arg Gly Thr Glu Thr Met Cys Leu Asp Leu Val
           115                 120                 125
   Gly Tyr Arg Glu Glu Asn Tyr Ala Gly Asn Ile Thr Tyr Asn Ile Lys
       130                 135                 140
   Gly Cys Val Ser Ser Cys Pro Leu Leu Thr Leu Ser Glu Arg Gly His
   145                 150                 155                 160
   Glu Gly Arg Lys Asn Asp Leu Lys Lys Val Glu Cys Arg Glu Ala Leu
                   165                 170                 175
   Lys Pro Ala Ser Ser Asp
               180
<210> 4
   <211> 182
   <212> PRT
   <213> Python sebae
<400> 4
   His Lys Cys Glu Ile Cys His Gly Phe Gly Asp Asp Cys Asp Gly Tyr
   1               5                   10                  15
   Gln Glu Glu Cys Pro Ser Pro Glu Asp Lys Cys Gly Lys Ile Leu Ile
               20                  25                  30
   Asp Ile Ala Leu Ala Pro Val Ser Phe Arg Ala Thr His Lys Asn Cys
           35                  40                  45
   Phe Ser Ser Ser Ile Cys Lys Leu Gly Arg Val Asp Ile His Val Trp
       50                  55                  60
   Glu Gly Val Tyr Ile Arg Gly Arg Thr Asn Cys Cys Asp Asn Asp Gln
   65                  70                  75                  80
   Cys Glu Asp Gln Pro Leu Pro Gly Leu Pro Leu Ser Leu Lys Asn Gly
                   85                  90                  95
   Leu Tyr Cys Pro Gly Ala Phe Gly Ile Phe Thr Glu Tyr Ser Thr Glu
               100                 105                 110
   His Glu Val Lys Cys Arg Gly Thr Glu Thr Met Cys Leu Asp Leu Val
           115                 120                 125
   Gly Tyr Arg Glu Glu Asn Tyr Val Gly Asn Ile Thr Tyr Asn Ile Lys
       130                 135                 140
   Gly Cys Val Ser Ser Cys Pro Leu Val Thr Leu Ser Glu Arg Gly His
   145                 150                 155                 160
   Glu Gly Arg Lys Asn Asp Leu Lys Lys Val Glu Cys Arg Glu Ala Leu
                   165                 170                 175
   Lys Tyr Glu Ser Ser Asp
               180
<210> 5
   <211> 182
   <212> PRT
   <213> Artificial Sequence
<220>
   <223> preparate mediante tecniche di biologia molecolare a partire dalla sequenza amminoacidica delle proteine denominate P3 e P4
<400> 5
   His Lys Cys Glu Ile Cys His Gly Phe Gly Asp Asp Cys Asp Gly Tyr
   1               5                   10                  15
   Glu Glu Glu Cys Pro Ser Pro Glu Asp Gln Cys Gly Lys Ile Leu Ile
               20                  25                  30
   Asp Ile Ala Leu Ala Pro Leu Ser Phe Arg Ala Thr His Lys Asn Cys
           35                  40                  45
   Phe Ser Ser Ser Ile Cys Lys Leu Gly Arg Val Asp Ile His Val Trp
       50                  55                  60
   Asp Gly Val Tyr Met Arg Gly Arg Thr Asn Cys Cys Asp Asn Asp Gln
   65                  70                  75                  80
   Cys Glu Asp Glu Pro Leu Pro Gly Leu Pro Leu Ser Leu Lys Asn Gly
                   85                  90                  95
   Leu Tyr Cys Pro Gly Ala Phe Gly Ile Phe Thr Glu Asp Ser Thr Glu
               100                 105                 110
   His Glu Val Lys Cys Arg Gly Thr Glu Thr Met Cys Leu Asp Leu Val
           115                 120                 125
   Gly Tyr Arg Glu Glu Asn Tyr Ala Gly Asn Ile Thr Tyr Asn Ile Lys
       130                 135                 140
   Gly Cys Val Ser Ser Cys Pro Leu Ile Thr Leu Ser Glu Arg Gly His
   145                 150                 155                 160
   Glu Gly Arg Lys Asn Asp Leu Lys Lys Val Glu Cys Arg Glu Ala Leu
                   165                 170                 175
   Lys Pro Ala Ser Ser Asp
               180
<210> 6
   <211> 182
   <212> PRT
   <213> Artificial Sequence
<220>
   <223> preparate mediante tecniche di biologia molecolare a partire dalla sequenza amminoacidica delle proteine denominate P3 e P4
<400> 6
   His Lys Cys Glu Ile Cys His Gly Phe Gly Asp Asp Cys Asp Gly Tyr
   1               5                   10                  15
   Glu Glu Glu Cys Pro Ser Pro Glu Asp Gln Cys Gly Lys Ile Leu Ile
               20                  25                  30
   Asp Ile Ala Leu Ala Pro Leu Ser Phe Arg Ala Thr His Lys Asn Cys
           35                  40                  45
   Phe Ser Ser Ser Ile Cys Lys Leu Gly Arg Val Asp Ile His Val Trp
       50                  55                  60
   Glu Gly Val Tyr Ile Arg Gly Lys Thr Asn Cys Cys Asp Asn Asp Gln
   65                  70                  75                  80
   Cys Glu Asp Glu Pro Leu Pro Gly Leu Pro Leu Ser Leu Lys Asn Gly
                   85                  90                  95
   Leu Tyr Cys Pro Gly Ala Phe Gly Ile Phe Thr Glu Tyr Ser Thr Glu
               100                 105                 110
   His Glu Val Lys Cys Arg Gly Thr Glu Thr Met Cys Leu Asp Leu Val
           115                 120                 125
   Gly Tyr Arg Glu Glu Asn Tyr Val Gly Asn Ile Thr Tyr Asn Ile Lys
       130                 135                 140
   Gly Cys Val Ser Ser Cys Pro Leu Ile Thr Leu Ser Glu Arg Gly His
   145                 150                 155                 160
   Glu Gly Arg Lys Asn Asp Leu Lys Lys Val Glu Cys Arg Glu Ala Leu
                   165                 170                 175
   Lys Tyr Glu Ser Ser Asp
               180
<210> 7
   <211> 35
   <212> PRT
   <213> Artificial Sequence
<220>
   <223> oligonucleotide sintentico
<400> 7
   Gly Ala Ala Thr Thr Cys Cys Ala Thr Ala Ala Ala Thr Gly Thr Gly
   1               5                   10                  15
   Ala Ala Ala Thr Thr Thr Gly Thr Cys Ala Thr Gly Gly Cys Thr Thr
               20                  25                  30
   Thr Gly Gly
   35
<210> 8
   <211> 48
   <212> PRT
   <213> Artificial Sequence
<220>
   <223> oligonucleotide sintentico
<400> 8
   Gly Cys Gly Gly Gly Cys Cys Gly Cys Thr Thr Ala Ala Thr Cys Ala
   1 5 10 15
   Gly Ala Gly Gly Ala Thr Gly Cys Ala Gly Gly Thr Thr Thr Cys Ala
   20 25 30
   Ala Gly Gly Cys Thr Thr Cys Cys Cys Thr Ala Cys Ala Thr Thr Cys
   35 40 45
<210> 9
   <211> 47
   <212> PRT
   <213> Artificial Sequence
<220>
   <223> oligonucleotide sintentico
<400> 9
   Gly Cys Gly Gly Cys Cys Gly Cys Thr Thr Ala Ala Thr Cys Ala Gly
   1 5 10 15
   Ala Gly Gly Ala Thr Thr Cys Ala Thr Ala Thr Thr Thr Cys Ala Ala
   20 25 30
   Gly Gly Cys Thr Thr Cys Cys Cys Thr Ala Cys Ala Thr Thr Cys
   35 40 45

## Description

### Field of the invention

The present invention relates to the field of products with pharmacological activity and more specifically anti-tumor activity.

### State of the art

Chemotherapeutics are classified according to their chemical structure or their biological activity (see "Pharmacological Basis of Therapeutics "by A. Goodman and M. Gilman, Pergamon Press, New York, 2005). In many cases, antitumor drugs act on cell cycle blocking cell growth.

The classification of chemotherapeutics includes also natural compounds or their derivatives (like taxol or vincristine) which can bind tubulin, thereby inhibiting mitosis.

Patent application WO 2011/001403 described a new protein characterized for its pro-apoptotic properties; said protein is a heterotetramer made of four subunits consisting of two different pairs of identical monomers.

In WO 02/47697 in the name of the same applicants similar factors extravcted from Python sebae blood are described and their anti-tumor cel activity and telomerase inhibition activity is reported.

More specifically, two of such monomers, identified as P1 in the above application, have sequence (SEQ ID N°1): while the other two, identified as P2, have sequence (SEQ. ID N°2):

Where the symbol P* refers to methylproline.

However, due to the importance of cancer chemotherapy, it is obvious the extreme usefulness of other compounds capable of selective pro-apoptotic action on cancer cells without affecting healthy cells.

### Short description of the figures

Figure 1 shows the effect of F5 on tumor growth in vivo.
Figure 2 shows the activity of F5 on expression of the active/cleaved form of caspase-3
Figure 3 shows the activity of P5, P6 and P5P6 proteins on expression of the active/cleaved form of caspase-3

### Summary of the invention

Proteins are described which exhibit pro-apoptotic and anti-telomerase activity on cancer cells and are useful for preparation of pharmaceutical formulations capable of inducing apoptosis of cancer cells and of cancer stem cells in a selective manner.

### Detailed description of the invention

Surprisingly, it has now been found that other proteins with a structure similar to the above mentioned two proteins, although carrying significant sequence divergence of key amino acids, show better activity than the initial proteins and also exhibit pro-apoptotic activity on cancer stem cells.

More specifically, the proteins according to the invention, hereinafter referred to as P3, P4, P5 and P6, carry the following amino acid sequences, respectively: and and

It can be observed (i.e., amino acids in bold and underlined) that P3 differs from P1 with respect to amino acids at positions 94, 132, 134 and 153; P4 differs from P2 with respect to amino acids at positions 26, 94, 109, 132, 144 and 136; P5 differs from P3 with respect to amino acids at positions 26, 39, 84 and 153 and finally P6 differs from P4 with respect to amino acids at positions 17, 26, 39, 72, 84 and 153.

P3 and P4 subunits, about 23 kDa each, interact non-covalently in 1:1 stoichiometric ratio to form a hetero-tetrameric structure existing in equilibrium with a hetero-hexameric structure.

Each subunit also contains a carbohydrate chain of about 3 kDa attached to the Asn138 residue and 16 cysteine residues forming eight disulfide bridges between them.

The two P3 and P4 subunits have high sequence similarity with only six amino acid substitutions identified between them (i.e., Glu17Gln, Asp65Glu, Met69IIe, Asp109Tyr, Ala136Val, Leu153Val).

Spectroscopic data indicate a very similar secondary structure of P3 and P4, with high beta sheet content. P3 and P4 separated by HPLC purification completely lose antitumor activity. This indicates that the activity is intrinsically associated with formation of a biologically active oligomeric complex.

The above defined P3 and P4 units were isolated from python serum as described below. Instead P5 and P6 units were prepared by means of molecular biology techniques having as starting point the amino acid sequence of proteins designated P3 and P4.

### Example 1

Isolation of the hetero-tetrameric structure in equilibrium with the hetero-hexameric structure composed by P3 and P4 units in 1:1 stoichiometric ratio.

Python serum, termed PSS, (100 µl) was diluted 1:5 with buffer consisting of 20 mM Tris-HCl, pH 7.5, 0.15 M NaCl, centrifuged for 2 minutes at 13000 g, filtered through a 0.45 µm filter and fractionated by ion exchange chromatography by use of a MonoQ column (0.6x6 cm) (GE-Helathcare). The column was equilibrated in 20 mM Tris-HCl, pH 7.5 buffer containing 0.15 M NaCl at a flow rate of 0.5 ml/min and eluted with a linear NaCl gradient from 0.15 to 1.0 M. The absorbance of the effluent was recorded at 280 nm. 1 ml fractions were collected and assayed for cytotoxic activity by the MTT assay (see below). Among collected fractions, only fraction F5 that eluted at about 0.43 M NaCl was found to be active. The material eluted in fraction F5 was collected and chemically characterized. In particular, the following analyses were performed:
1) determination of total protein content by bicinchoninic acid assay (BCA);
2) electrophoretic analysis in polyacrylamide gel in the presence of SDS according to Laemmli;
3) analytical gel-filtration chromatography in Superose-12 column (1 x 30 cm) (GE-Healthcare);
4) dynamic light scattering measurements by use of a Zetasizer Nano S instrument (Malvern);
5) determination of the amino acid sequence of P3 and P4 by tryptic fragmentation and subsequent high resolution mass analysis.

The results indicate that fraction F5 is composed of two subunits, P3 and P4, having the above amino acid sequence, and that these subunits are present in F5 in 1:1 stoichiometric ratio to form a non-covalent tetrameric complex existing in equilibrium with a hexameric complex.

### Biological tests

From a biological viewpoint, the fraction composed by the two peptide units P3 and P4, hereinafter referred to as F5, was tested for antitumor and pro-apoptotic properties by in vivo and in vitro studies.

In vivo, fraction F5 inhibits by 60% the volume of tumor growth in the A431 squamous cancer cell model following subcutaneous grafting in immunodeficient mice. F5 activity was compared with phosphate buffer (PBS) and/or with another python Sebae serum fraction not containing P3/P4 and therefore found to be inactive (subsequently termed F3).

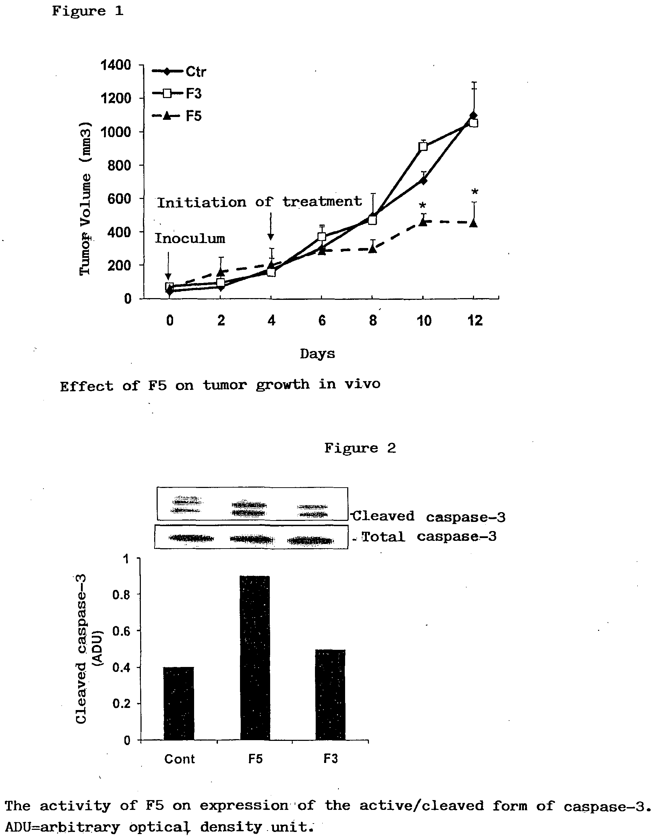

In short, squamous carcinoma cells A431 (10⁶/mouse) are implanted subcutaneously in immunodeficient Balb/c mice. F5 (50 µg/100µl) or F3 (50 µg/100µl) or vehicle (PBS, 100µl) are administered daily by injection nearby the tumor mass. Tumor volumes are reported as mean ± SD, n = 6, * P <0.05 vs. vehicle (Fig. 1).

In vitro, a cytotoxicity study has shown that F5 significantly inhibits survival of various types of tumor cells such as A431 (squamous carcinoma), MCF7 (breast cancer), and U373MG (glioblastoma) (Table 1). As above, F3 does not show cytotoxic activity.

**TABLE 1**

| **Sopravvivenza (% Cellule vive)** | | | | | **%Cellule morte/numero totale di cellule cortate** | | |
|---|---|---|---|---|---|---|---|
| | **A431** | **MCF-7** | **U373MG** | | **A431** | **MCF-7** | **U373MG** |
| **Cortrollo** | 100 | 100 | 100 | | 1.4±1.6 | 2.4±2 | 3.2±3 |
| **F3** | 90±10 | 87±15 | 94±12 | | 3±2.7 | 2.9±1.7 | 4.7±4 |
| **F5** | 63±4** | 53±3** | 58±5** | | 14±4* | 21±3.4* | 18±5* |
| Survival (% Viable cells) | | | | | % Dead cells / total cell counts | | |

Cell viability was assessed by trypan blue and MTT assays after 1 hour and 24 hours, respectively, from the treatment with F5 or F3 (50 µg/ml). In short, for the trypan blue assay, tumor cells in suspension were incubated for 1 hour with the two fractions, whereas for the MTT assay plated cells were incubated with the fractions for 24 hours. After incubation, trypan blue, a dye that does not penetrate viable cells, or MTT, a yellow tetrazole salt which is reduced to purple formazan in viable cells, is added to the cell culture. MTT data reported as % cell viability are the average of two experiments performed in triplicate. The data of trypan blue assay are reported as % dead cells on total cells counts. Control = DMEM +0.1% FCS. *p<0.05, **p< 0.01 vs control (Table 1).

Two experiments were performed to define the mechanism of action of F5: i. pro-apoptotic activity of F5 was evaluated by western blot detection of caspase-3; ii. activity of the telomerase enzyme was measured in tumor cells incubated with F5. In short, A431 tumor cells were exposed for 24 hours to F5 or F3 (50 µg/ml) and expression of intact or cleaved/active form of the pro-apoptotic enzyme caspase-3 was measured by western blotting. F5 promotes increased expression of the cleaved form of caspase-3, indicative of pro-apoptotic activity, while F3 shows no activity (Fig. 2). The bar graph represents a densitometric quantification of caspase 3 bands in Western blots.

It is well known that the activity of telomerase enzyme contributes to the control of cell immortalization via telomere-reconstruction. In fact, telomerase activity is increased in tumor cells but is drastically decreased in differentiated cells. Consequently certain (non-tumor specific) anticancer agents acting on telomerase are directed at inhibiting this activity to prevent telomere restoration and promote cell death. Tests of fraction F5 on telomerase activity were made by a method established in our laboratories (modified TRAP method, see Gelmini S. et al; Rapid, quantitative non isotopic assay for telomerase activity in human tumors. Clin Chem 44, 2133, 1998). The experiments investigated the direct effect of F5 on the activity of telomerase enzyme extracted from the CHP cell line (neuroblastoma line). F5 decreases telomerase activity in a concentration-dependent manner (F5 at 50 µg/100 µl reduces telomerase activity by 4.6 fold).

### Example 2

### Production of P5 and P6 units.

Liver biopsy was performed from python *sebae.* Total RNA was extracted from liver biopsy specimens by use of a specific kit for small amounts of tissue (Qiagen). Subsequently, RNA was reverse transcribed to obtain the complementary DNA (cDNA) strand. For this purpose, a method was used that involved the Superscript-II reverse transcriptase enzyme and hexanucleotides with random sequence. 1 µl of the cDNA thus obtained was amplified by *polymerase chain reaction* (PCR), according to the Qiagen protocol.

Selective amplification of cDNA sequences was carried out using the following synthetic oligonucleotide pairs:
GAATTCCATAAATGTGAAATTTGTCATGGCTTTGG (SEQ ID 7) GCGGGCCGCTTAATCAGAGGATGCAGGTTTCAAGGCTTCCCTACATTC (SEQ ID 8)
GAATTCCATAAATGTGAAATTTGTCATGGCTTTGG (SEQ ID 7) GCGGCCGCTTAATCAGAGGATTCATATTTCAAGGCTTCCCTACATTC (SEQ ID 9)

Said synthetic oligonucleotides were provided in purified form by MWG_Biotech, Germany.

PCR products were cloned into plasmid psk-Bluescript (Stratagene) [Alting-Mes et al. (1992) Methods Enzymol. 216, 483-495] thus obtaining the respective plasmid constructs that were then subjected to sequencing that resulted in the above P5 and P6 sequences.

Finally, by standard molecular biology techniques, the corresponding P5 and P6 recombinant proteins were expressed in *Escherichia coli* as fusion proteins containing N-terminal glutathione S-transferase (GST, 27 kDa) in order to obtain corresponding P5-GST and P6-GST proteins on which the biological tests were performed.

For this purpose, equimolar solutions of P5-GST and P6-GST were prepared in stoichiometric 1:1 ratio and their pro-apoptotic activity on human A431 tumor cells was tested by western blotting.

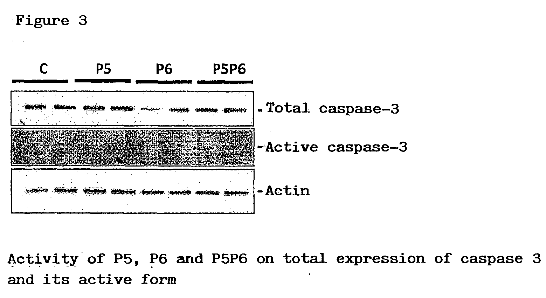

As it can be seen in Figure 3, P5-GST and P6-GST proteins induce apoptosis in cancer cells. The possibility to obtain bacterial recombinant P5-GST and P6-GST paves the way to large scale production of these protein for their future use in pharmaceutical preparations with antitumor activity.

## Claims

1. Protein with pro-apoptotic and anti-telomerase activity on cancer cells having the following amino acid sequence:

2. Protein with pro-apoptotic and anti-telomerase activity on cancer cells having the following amino acid sequence:

3. Protein according to claims 1 and 2 wherein each said protein contains a carbohydrate chain of about 3 kDa attached to the Asn138 residue and 16 cysteine residues forming eight disulfide bridges between them.

4. Protein with pro-apoptotic and antitelomerase activity on cancer cells having the following amino acid sequence:

5. Protein with pro-apoptotic and anti-telomerase activity on cancer cells having the following amino acid sequence:

6. Protein complex consisting of the two proteins according to claims 1 and 2 which interact non-covalently in 1:1 stoichiometric ratio to form a hetero-tetrameric structure in equilibrium with a hetero-hexameric structure.

7. Equimolar solutions of the two proteins according to claims 4 and 5 in 1:1 stoichiometric ratio.

8. Use of a protein according to claims 1 -5, the protein complex of claim 6 or the solution of claim 7 for preparation of pharmaceutical formulations with selective ability to induce apoptosis in cancer cells.

9. Use according to claim 8 wherein said cancer cells are cancer cells selected from: A431 squamous cell carcinoma, MCF7 breast cancer, and U373MG glioblastoma.

10. Use of a protein according to claims 1 - 5, the protein complex of claim 6 or the solution of claim 7 for preparation of pharmaceutical formulations with selective antitelomerase activity on cancer cells and cancer stem cells.

11. Use according to claims 8-10 wherein said pharmaceutical formulations have inhibitory activity on tumor vasculature.

## Patentansprüche

1. Protein mit pro-apoptotischer und Anti-Telomerase-Aktivität an Krebszellen, das die folgende Aminosäure-Sequenz aufweist:

2. Protein mit pro-apoptotischer und Anti-Telomerase-Aktivität an Krebszellen, das die folgende Aminosäure-Sequenz aufweist:

3. Protein nach den Ansprüchen 1 und 2, wobei jedes der Proteine eine Kohlenhydrat-Kette von etwa 3 kDa an den Rest Asn138 gebunden und 16 Cystein-Reste enthält, die acht Disulfid-Brücken zwischen ihnen bilden.

4. Protein mit pro-apoptotischer und Anti-Telomerase-Aktivität an Krebszellen, das die folgende Aminosäure-Sequenz aufweist:

5. Protein mit pro-apoptotischer und Anti-Telomerase-Aktivität an Krebszellen, das die folgende Aminosäure-Sequenz aufweist:

6. Protein-Komplex, bestehend aus zwei Proteinen gemäß den Ansprüchen 1 und 2, die nicht-kovalent in einem stöchiometrischen 1 : 1 - Verhältnis unter Bildung einer hetero-tetrameren Struktur im Gleichgewicht mit einer hetero-hexameren Struktur wechselwirken.

7. Equimolare Lösungen der zwei Proteine nach den Ansprüchen 4 und 5 in einem stöchiometrischen 1 : 1 - Verhältnis.

8. Verwendung eines Proteins nach den Ansprüchen 1 bis 5, des Protein-Komplexes nach Anspruch 6 oder der Lösung nach Anspruch 7 zum Herstellen pharmazeutischer Formulierungen mit selektivem Vermögen zum Induzieren von Apoptose in Krebszellen.

9. Verwendung nach Anspruch 8, wobei die Krebszellen Krebszellen sind, die gewählt sind aus A431-Plattenepithelkarzinom, MCF7-Brustkrebs und U373MG-Glioblastom.

10. Verwendung eines Proteins nach den Ansprüchen 1 bis 5, des Protein-Komplexes nach Anspruch 6 oder der Lösung nach Anspruch 7 zum Herstellen pharmazeutischer Formulierungen mit selektiver Antitelomerase-Aktivität an Krebszellen und Krebs-Stammzellen.

11. Verwendung nach den Ansprüchen 8 bis 10, wobei die pharmazeutischen Formulierungen Inhibitor-Aktivität an Krebs-Vaskulatur haben.

## Revendications

1. Protéine ayant une activité pro-apoptotique et anti-télomérase sur des cellules cancéreuses possédant la séquence d'acides aminés suivants :

2. Protéine ayant une activité pro-apoptotique et anti-télomérase sur des cellules cancéreuses possédant la séquence d'acides aminés suivants :

3. Protéine selon les revendications 1 et 2, où chaque dite protéine contient une chaîne glucidique d'environ 3 kDa attachée au résidu Asn138 et 16 résidus cystéine formant huit ponts disulfure entre eux.

4. Protéine ayant une activité pro-apoptotique et anti-télomérase sur des cellules cancéreuses possédant la séquence d'acides aminés suivants :

5. Protéine ayant une activité pro-apoptotique et anti-télomérase sur des cellules cancéreuses possédant la séquence d'acides aminés suivants :

6. Complexe protéique consistant en les deux protéines selon les revendications 1 et 2 qui interagissent de façon non covalente selon un rapport stoechiométrique de 1:1 afin de former une structure hétérotétramérique en équilibre avec une structure hétérohexamérique.

7. Solutions équimolaires des deux protéines selon les revendications 4 et 5 selon un rapport stoechiométrique de 1:1.

8. Utilisation d'une protéine selon les revendications 1-5, du complexe protéique selon la revendication 6 ou de la solution selon la revendication 7 pour la préparation de formulations pharmaceutiques ayant une aptitude sélective à induire l'apoptose dans des cellules cancéreuses.

9. Utilisation selon la revendication 8, dans laquelle lesdites cellules cancéreuses sont des cellules cancéreuses sélectionnées parmi : un carcinome à cellules squameuses A431, un cancer du sein MCF7, et un glioblastome U373MG.

10. Utilisation d'une protéine selon les revendications 1 à 5, du complexe protéique selon la revendication 6 ou de la solution selon la revendication 7 pour la préparation de formulations pharmaceutiques ayant une activité anti-télomérase sélective sur des cellules cancéreuses et des cellules souches cancéreuses.

11. Utilisation selon les revendications 8 à 10, dans laquelle lesdites formulations pharmaceutiques présentent une activité inhibitrice sur le système vasculaire d'une tumeur.
